# EUROPÄISCHE PATENTANMELDUNG

(11) **EP 0 998 937 A2**
(43) Veröffentlichungstag der Anmeldung: **10.05.2000**
(21) Anmeldenummer: 99121568.2
(22) Anmeldetag: 29.10.1999
(51) Int. Cl.: A61K 33/00, A61K 31/19, A61P 7/08

(54) **Verfahren zur Herstellung einer Lösung, insbesondere einer Dialyse- oder Infusionslösung**

(30) Priorität: 04.11.1998 DE 19850830
(71) Anmelder: Fresenius Medical Care Deutschland GmbH, 61350 Bad Homburg v.d.H. (DE)
(72) Erfinder: Knerr, Thomas, Dr., 66606 St. Wendel (DE); Backhaus, Wendelin, Dipl.-Phys., 35789 Weilmünster (DE)
(74) Vertreter: Laufhütte, Dieter, Dr.-Ing.

(57) **Zusammenfassung**

Die vorliegende Erfindung betrifft ein Verfahren zur Herstellung einer Lösung, insbesondere einer Dialyse- oder Infusionslösung. Die Erfindung betrifft ferner eine Lösung, insbesondere eine Dialyse- oder Infusionslösung, sowie ein Vorlagesystem zur Herstellung einer derartigen Lösung. Nach dem erfindungsgemäßen Verfahren wird in einem ersten Vorlagebehältnis eine sauer reagierende Komponente, die eine feste Säure und/oder ein festes Salz umfaßt, die wasserlöslich und nicht hygroskopisch sind, in Wasser gelöst und mit einer in einem zweiten Vorlagebehältnis befindlichen alkalischen bicarbonathaltigen Lösung gemischt. Dabei werden die Mengenverhältnisse derart gewählt, daß je Liter der fertigen Lösung der Gehalt an Säure und/oder Salz 6 mmol und an Bicarbonat 40 mmol nicht übersteigt.

## Beschreibung

Die vorliegende Erfindung betrifft ein Verfahren zur Herstellung einer Lösung, insbesondere einer Dialyse- oder Infusionslösung. Die Erfindung betrifft ferner eine Lösung, insbesondere eine Dialyse- oder Infusionslösung, sowie ein Vortagesystem zur Herstellung einer derartigen Lösung.

Für den Einsatz bei der Hämodialyse wie auch bei der Bauchfelldialyse (Peritonealdialyse) müssen Dialyselösungen bereitgestellt werden, die möglichst physiologisch sind, d.h. einen pH-Wert von etwa 7,4 aufweisen, wichtige Elektrolyten enthalten und ferner ein Puffersystem aufweisen, das physiologisch ist und sich zur Einstellung des gewünschten pH-Wertes eignet. Da Bicarbonat auch den physiologischen Puffer des Blutes darstellt, wird als Puffersystem im allgemeinen Bicarbonat verwendet.

Ein wesentlicher Nachteil des Bicarbonat-Puffersystems besteht darin, daß dieses bei geringen pH-Werten durch Ausgasen CO₂ abgibt und somit entsprechend instabil ist. Um das Ausgasen zu vermeiden, wird in der Praxis der pH-Wert möglichst hoch gehalten, wodurch sich bei der Anwesenheit entsprechender Kationen das Problem ergibt, daß diese mit dem bei hohen pH-Werten vorliegenden Carbonat ausfallen. Dies gilt beispielsweise für die üblicherweise in Dialyselösungen vorhandenen Calcium- und Magnesiumionen.

Um dennoch den gewünschten physiologischen pH-Wert der Dialyselösung einzustellen, wird mit einem zweiten Puffersystem gepuffert, das in der Regel Acetat oder auch andere metabolisierbare Säuren aufweist. Um einerseits das Ausgasen der bicarbonathaltigen Lösung zu verhindern und andererseits das vor allem bei hohen pH-Werten auftretende Ausfallen von Carbonaten zu vermeiden, werden die fertigen Dialyselösungen üblicherweise aus zwei getrennt aufbewahrten Einzellösungen hergestellt. Dabei befindet sich in einem Behältnis eine bicarbonathaltige Lösung und in einem anderen Behältnis das zweite, saure Puffersystem. Die saure Komponente enthält meist auch die für die Dialyse benötigten Elektrolyten, wie z.B. NaCl oder KCl.

Zur Herstellung der anwendungsfertigen Lösung werden die genannten Einzellösungen gemischt, wobei die Gefahr der Carbonatausfällung nur von geringer Bedeutung ist, da die Verweildauer der fertigen Dialyselösung im Leitungssystem des Dialysegerätes nur in der Größenordnung von etwa einer Minute liegt.

Die bicarbonathaltige sowie die die saure Komponente enthaltende Einzellösung werden üblicherweise in Form von Konzentratkanistern angeliefert und dann vor Ort gemischt und mit Reinstwasser auf das gewünschte Konzentrationsniveau verdünnt. Das Konzentrat wird in handelsüblichen Behältern mit einem Fassungsvermögen von ca. 10 l bereitgestellt, was nicht nur erhebliche Probleme beim Handling der Behälter durch das behandelnde Personal mit sich bringt, sondern aufgrund der Tatsache, daß unterschiedliche Konzentrate eingesetzt werden müssen auch einen erheblichen Aufwand für die Lagerhaltung der Konzentrate bedeutet.

Die EP 526 754 beschreibt die Herstellung einer derartigen Dialyselösung durch Mischen zweier Einzellösungen, wobei eine der Einzellösungen eine bicarbonathaltige alkalische Lösung und die andere eine bicarbonatfreie saure Lösung darstellt. Die saure Lösung enthält metabolisierbare organische Säuren, während die wesentlichen Bestandteile der alkalischen Einzellösung Alkalibicarbonat und Alkalicarbonat sind.

Um das Problem der aufwendigen Handhabung der Konzentratbehälter zu vermeiden, ist bereits vorgeschlagen worden, die für die Dialyse notwendigen Stoffe als Festsubstanzen anzuliefern und vor Ort die notwendigen Konzentrate durch Lösen der Feststoffe herzustellen. Dabei ergibt sich das Problem, daß die Vorlage fester Säuren schwierig ist, da diese im allgemeinen zu hygroskopisch sind oder insbesondere bei hohen Bicarbonatkonzentrationen für eine optimale pH-Wert-Einstellung ungeeignet sind.

Es ist daher die Aufgabe der vorliegenden Erfindung eine Lösung, insbesondere eine Infusions- oder Dialyselösung, bereitzustellen, die nur einen geringen Herstellaufwand erfordert und physiologisch verträglich ist.

Diese Aufgabe wird durch die Merkmale der Ansprüche 1, 8 und 12 gelöst. Bei dem erfindungsgemäßen Verfahren zur Herstellung einer Lösung, insbesondere einer Dialyse- oder Infusionslösung, wird in einem ersten Vorlagebehältnis eine sauer reagierende Komponente, die eine feste Säure und/oder ein festes Salz umfaßt, die wasserlöslich und nicht hygroskopisch sind, in Wasser gelöst und mit einer in einem zweiten Vorlagebehältnis befindlichen alkalischen bicarbonathaltigen Lösung gemischt, wobei die Mengenverhältnisse derart gewählt werden, daß je Liter der fertigen Lösung der Gehalt an Säure und/oder Salz 6 mmol und an Bicarbonat 40 mmol nicht übersteigt.

Aufgrund der Beschaffenheit der verwendeten Säure bzw. des verwendeten Salzes als Feststoffe ist die Zubereitung einer entsprechenden Lösung unproblematisch.

Nach dem Lösen der Säure bzw. des Salzes in dem ersten Vorlagebehältnis erfolgt die Mischung mit der bicarbonathaltigen Lösung, wobei trotz eines verhältnismäßig hohen Bicarbonatgehaltes von bis zu 40 mmol je Liter fertiger Lösung ein Gehalt an Säure und/oder Salz von bis zu 6 mmol je Liter fertiger Lösung ausreicht, um eine physiologisch verträgliche Lösung bereitzustellen.

In weiterer Ausgestaltung der vorliegenden Erfindung ist vorgesehen, daß die Mischung aus der die sauer reagierende Komponente enthaltenden Lösung sowie der alkalischen bicarbonathaltigen Lösung vor der Verwendung mit Wasser verdünnt wird. Entsprechend wird aus der als Feststoff vorliegenden Säure bzw. dem Salz im Vorlagebehältnis zunächst ein Konzentrat gebildet und vor oder nach der Mischung mit der alkalischen bicarbonathaltigen Lösung auf den gewünschten Wert mit Wasser verdünnt.

In weiterer Ausgestaltung der vorliegenden Erfindung ist vorgesehen, daß das Bicarbonat als Feststoff eingesetzt wird und die alkalische bicarbonathaltige Lösung durch Lösen des Feststoffes hergestellt wird. Eine derartige Vorgehensweise weist den Vorteil auf, daß als Ausgangsstoffe zur Herstellung der entsprechenden Lösungen nur Feststoffe eingesetzt werden, was sowohl die Bereitstellung als auch die Lagerhaltung vereinfacht. Erfindungsgemäß wird somit aus einem bicarbonathaltigen Feststoff in dem zweiten Vorlagebehältnis die alkalische bicarbonathaltige Lösung hergestellt.

Besonders vorteilhaft ist es, wenn der Bicarbonatgehalt je Liter der fertigen Lösung im Bereich zwischen 32 und 35 mmol liegt, um metabolische Acidose von niereninsuffizienten Patienten zu kompensieren.

Gemäß einer bevorzugten Ausgestaltung der vorliegenden Erfindung liegt der Gehalt an Säure und/oder Salz je Liter fertiger Lösung bei ≤ 6 mval, vorzugsweise bei 2-4 mval. Trotz verhältnismäßig hoher Bicarbonatkonzentrationen von bis zu 40 mmol/l ist ein Gehalt an Säure und/oder Salz je Liter fertiger Lösung von unter 6 mval, vorzugsweise von 2-4 mval ausreichend, um die fertige Lösung auf den physiologischen pH-Wert von ca. 7,4 abzupuffern.

In weiterer Ausgestaltung der vorliegenden Erfindung ist vorgesehen, daß als Säure und Salz Zitronensäure, Isozitronensäure, 2-Oxoglutarsäure, Bernsteinsäure, Fumarsäure, Äpfelsäure und/oder Oxalessigsäure und deren Salze eingesetzt werden. Als Säure und Salz können ferner Hydrochloride der Aminosäuren und deren Salze eingesetzt werden.

Die vorliegende Erfindung betrifft ferner eine Lösung, insbesondere eine Dialyse- oder Infusionslösung, mit einer alkalisch reagierenden bicarbonathaltigen Komponente sowie einer eine Säure und/oder ein Salz umfassenden sauer reagierenden Komponente, wobei die Säure und das Salz unter Normalbedingungen als Feststoffe vorliegen, wasserlöslich und nicht hygroskopisch sind, und wobei der Gehalt an Salz und/oder Säure je Liter fertiger Lösung 6 mmol und an Bicarbonat 40 mmol nicht übersteigt.

Besonders vorteilhaft ist es, wenn der Bicarbonatgehalt je Liter fertiger Lösung im Bereich zwischen 32 und 35 mmol liegt. Der Gehalt an Säure und/oder Salz je Liter fertiger Lösung kann bei ≤ 6 mval, vorzugsweise bei 2-4 mval liegen.

Besonders vorteilhaft ist es, wenn als Säure bzw. Salz Zitronensäure, Isozitronensäure, 2-Oxoglutarsäure, Bernsteinsäure, Fumarsäure, Äpfelsäure und/oder Oxalessigsäure und deren Salze eingesetzt werden. Ebenso können Hydrochloride der Aminosäuren und deren Salze eingesetzt werden.

Die vorliegende Erfindung betrifft ferner ein Vorlagesystem zur Herstellung einer Lösung, insbesondere einer Dialyse- oder Infusionslösung, mit wenigstens zwei Vorlagebehältnissen, wobei in einem der Vorlagebehältnisse eine sauer reagierende und in dem anderen Vorlagebehältnis eine alkalisch reagierende bicarbonathaltige Komponente aufgenommen ist, wobei die sauer reagierende Komponente eine feste Säure und/oder ein festes Salz aufweist, die wasserlöslich und nicht hygroskopisch sind und die eine derartige Pufferkapazität aufweisen, daß bei einer Konzentration der Säure und/oder des Salzes von ≤ 6 mmol je Liter fertiger Lösung und einer Bicarbonat-Konzentration von bis zu 40 mmol je Liter fertiger Lösung ein physiologischer pH-Wert der fertigen Lösung einstellbar ist.

Besonders vorteilhaft ist es, wenn die Säure und das Salz gemäß der in Anspruch 6 und 7 genannten Substanzen ausgewählt sind.

In weiterer Ausgestaltung der Erfindung ist vorgesehen, daß die alkalisch reagierende bicarbonathaltige Komponente in dem Vorlagebehältnis als Feststoff vorliegt.

Gemäß einer bevorzugten Ausgestaltung der vorliegenden Erfindung sind drei Vorlagebehältnisse vorgesehen, wobei das erste Vorlagebehältnis die sauer reagierende Komponente, das zweite Vorlagebehältnis die alkalisch reagierende bicarbonathaltige Komponente und das dritte Vorlagebehältnis Zusätze enthält.

Besonders vorteilhaft ist es, wenn alle Komponenten und Zusätze in den Vorlagebehältnissen als Feststoffe vorliegen. Die Zusätze können NaCl umfassen.

Weitere Einzelheiten und Vorteile der vorliegenden Erfindung werden anhand eines Ausführungsbeispiels näher erläutert.

Die nachfolgende Tabelle zeigt die pH-Werte einer fertigen Dialyselösung, bei der als feste Säure Bernsteinsäure verwendet wurde. Die Bernsteinsäure wurde in Wasser gelöst und eine 0,15 molare Bernsteinsäurelösung hergestellt. Dieses Konzentrat wurde anschließend mit einer 1 molaren Natriumhydrogencarbonatlösung gemischt und mit der angegebenen Menge an Wasser verdünnt.

| Bicarbonat-Succinat Misch pH-Werte | | | | | |
|---|---|---|---|---|---|
| NaHCO₃ | Wasser | Bernsteinsäure | 1. Versuch | 2. Versuch | 3. Versuch |
| 35 ml | 957 ml | 8 ml | 7,42 | 7,39 | 7,41 |
| 35 ml | 955 ml | 10 ml | 7,26 | 7,27 | 7,27 |
| 35 ml | 953 ml | 12 ml | 7,16 | 7,17 | 7,16 |
| 35 ml | 951 ml | 14 ml | 7,08 | 7,09 | 7,08 |
| 40 ml | 952 ml | 8 ml | 7,46 | 7,47 | 7,48 |
| 40 ml | 950 ml | 10 ml | 7,34 | 7,35 | 7,32 |
| 40 ml | 948 ml | 12 ml | 7,24 | 7,25 | 7,24 |
| 40 ml | 946 ml | 14 ml | 7,14 | 7,16 | 7,19 |

Die Tabelle zeigt, daß Bernsteinsäure bereits in einer Konzentration von beispielsweise 1,5 mmol/l bezogen auf die fertige Anwendungslösung ausreicht, um trotz der verhältnismäßig hohen Natriumhydrogencarbonatkonzentration einen physiologischen pH-Wert einzustellen.

Die Verwendung von Bernsteinsäure gemäß dem vorliegenden Ausführungsbeispiel bzw. der anderen erfindungsgemäßen Säuren bzw. Salze ist nicht nur auf Dialyselösungen zur Hämodialyse beschränkt, sondern ist auch bei der Herstellung von Peritonealdialyselösungen möglich.

Das erfindungsgemäße Vorlagesystem zur Herstellung einer Lösung, insbesondere einer Infusions- oder Dialyselösung, kann auch aus drei Vorlagebehältnissen bestehen, wobei vorteilhaft alle Vorlagebehältnisse Trockenkonzentrate enthalten. Dabei kann beispielsweise in einem ersten Vorlagebehältnis die sauer reagierende Komponente, beispielsweise Bernsteinsäure, in einem zweiten Vorlagebehältnis Natriumhydrogencarbonat und in einem dritten Vorlagebehältnis Natriumchlorid vorgelegt werden. Das erste Vorlagebehältnis kann ferner ein Gemisch der Minorbestandteile, wie z.B. Kaliumchlorid, aufweisen. Aus dem Natriumhydrogencarbonat und dem Natriumchlorid werden an der Dialysemaschine jeweils gesättigte Lösungen bereitet, die im weiteren Verlauf bis zur gebrauchsfertigen Lösung verdünnt werden. Die sauer reagierende Komponente, wie z.B. Bernsteinsäure, soll jedoch im Sinne eines batch-Ansatzes in einem festen Volumen Wasser gelöst werden und dann ebenfalls als definiertes Konzentrat weiterverarbeitet werden. Dabei ist von Bedeutung, mit welcher Geschwindigkeit sich die Säure bzw. das Salz in Wasser löst.

Das Konzentrat ist beispielsweise etwa 110-fach, d.h. 1 Liter des Konzentrats der sauer reagierenden Komponente werden mit 109 Litern Wasser bzw. Salzlösung zur anwendungsbereiten Lösung gemischt. Wird Bernsteinsäure verwendet, sind 0,165 mol bzw. 19,47 g Bernsteinsäure in einem Liter Konzentrat zu lösen.

Die Löslichkeit von Bernsteinsäure ist zwar prinzipiell ausreichend, doch insbesondere die Lösungsgeschwindigkeit ist sehr gering. Ferner kommt es aufgrund der speziellen Eigenart der Bernsteinsäure-Kristalle zu einer Flotation eines Teils des Materials, der sich dadurch aus der Durchmischungszone entfernt. Die gleichzeitige Anwesenheit der anderen Salze und von Glucose setzt die Löslichkeit und die Lösungsgeschwindigkeit der Bernsteinsäure weiter herab.

Es ist daher vorteilhaft die Lösungsgeschwindigkeit von Bernsteinsäure durch Teilneutralisation mittels einer Base zu erhöhen. Dazu wird ein Äquivalent Bernsteinsäure in einem möglichst kleinen Volumen von Wasser gegebenenfalls in der Wärme gelöst und mit 0,05-0,5 (bevorzugt 0,25) Äquivalenten Base versetzt. Das vollständig gelöste Produkt wird einem gängigen Trocknungsprozeß (Sprühtrocknung, Gefriertrocknung etc.) unterworfen und anschließend homogenisiert. Bevorzugte Basen sind Natronlauge bzw. Natriumhydroxid, Natriumcarbonat und Natriumbicarbonat, da mit diesen Basen die physiologisch unbedenklichen Natriumsalze der Bernsteinsäure gebildet werden. Da der die sauer reagierende Komponente enthaltende Vortagebehälter Kalium-, Magnesium- und Calciumsalze in entsprechenden Mengen enthalten kann, sind jedoch auch die Hydroxide, Oxide, Hydrogencarbonate und Carbonate dieser Kationen als Basen geeignet.

Die Erhöhung der Lösungsgeschwindigkeit wird durch das folgende Beispiel verdeutlicht.

0,2 mol Bernsteinsäure und 0,1 mol Natriumhydrogencarbonat werden in einem Becherglas mit 100 ml Wasser versetzt. Die Lösung wird gerührt und kurz erhitzt, bis die ganze Substanz in Lösung gegangen ist. Anschließend wird im Trockenschrank bei 105°C bis zur Gewichtskonstanz (48 h) getrocknet. Das amorph kristalline Produkt wird im Mörser homogenisiert.

Die folgende Tabelle zeigt die Lösungsgeschwindigkeit von Bernsteinsäure und des erhaltenen Bernsteinsäure-Natriumhydrogencarbonat-Adduktes.

| Lösungsgeschwindigkeit von Bernsteinsäure und Bernsteinsäure-NaHCO₃-Addukt in Gegenwart von 121 g/l Glucose-Hydrat | | | | | |
|---|---|---|---|---|---|
| Bernsteinsäure | Konzentration | Zeit bis zu klarer Lösung [s] | | | Mittelwert |
| 12,98 g/l ≅ 0,11 mol/l | 0,22 meq/l | 200 | 210 | 220 | 210 |
| 19,47 g/l ≅ 0,165 mol/l | 0,33 meq/l | 230 | 250 | 270 | 250 |
| 25,96 g/l ≅ 0,22 mol/l | 0,44 meq/l | 400 | 375 | 300 | 358 |
| 38,94 g/l ≅ 0,33 mol/l | 0,66 meq/l | 510 | 420 | 450 | 460 |
| 58,41 g/l ≅ 0,495 mol/l | 0,99 meq/l | 900 | > 1080 | 960 | > 900 |
| Bernsteinsäureaddukt ≅ freie Bernsteinsäure | Konzentration | Zeit bis zu klarer Lösung [s] | | | Mittelwert |
| 28,38 g/l ≅ 0,11 mol/l | 0,33 meq/l | 200 | 210 | 170 | 193 |
| 56,76 g/l ≅ 0,22 mol/l | 0,66 meq/l | 260 | 230 | 240 | 243 |
| 85,14 g/l ≅ 0,33 mol/l | 0,99 meq/l | 390 | 400 | 360 | 383 |

Die nachfolgende Grafik verdeutlicht die Beschleunigung der Lösungsgeschwindigkeit, wobei die x-Achse auf Säureäquivalente pro Liter Lösung skaliert ist. Im relevanten Konzentrationsbereich ist die Lösungsgeschwindigkeit des partiellen Natriumsalzes um 20-40 % größer als die der reinen Säure.

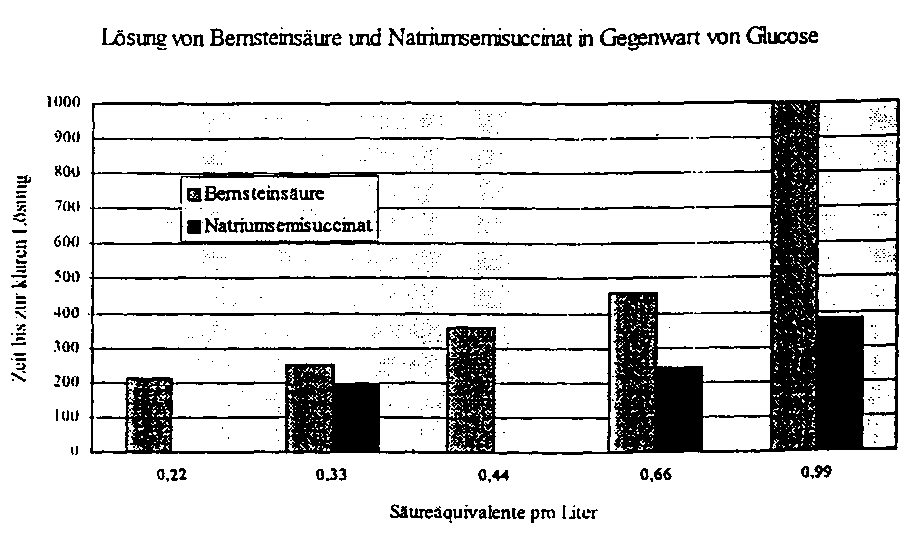

## Patentansprüche

1. Verfahren zur Herstellung einer Lösung, insbesondere einer Dialyse- oder Infusionslösung, bei dem in einem ersten Vorlagebehältnis eine sauer reagierende Komponente, die eine feste Säure und/oder ein festes Salz umfaßt, die wasserlöslich und nicht hygroskopisch sind, in Wasser gelöst und mit einer in einem zweiten Vorlagebehältnis befindlichen alkalischen bicarbonathaltigen Lösung gemischt wird, wobei die Mengenverhältnisse derart gewählt werden, daß je Liter der fertigen Lösung der Gehalt an Säure und/oder Salz 6 mmol und an Bicarbonat 40 mmol nicht übersteigt.

2. Verfahren nach Anspruch 1, dadurch gekennzeichnet, daß die Mischung aus der die sauer reagierende Komponente enthaltenden Lösung sowie der alkalischen bicarbonathaltigen Lösung vor der Verwendung mit Wasser verdünnt wird.

3. Verfahren nach Anspruch 1 oder 2, dadurch gekennzeichnet, daß das Bicarbonat als Feststoff eingesetzt wird und die alkalische bicarbonathaltige Lösung durch Lösen des Feststoffes hergestellt wird.

4. Verfahren nach einem oder mehreren der Ansprüche 1 bis 3, dadurch gekennzeichnet, daß der Bicarbonatgehalt je Liter der fertigen Lösung im Bereich zwischen 32 und 35 mmol liegt.

5. Verfahren nach einem oder mehreren der Ansprüche 1 bis 4, dadurch gekennzeichnet, daß der Gehalt an Säure und/oder Salz je Liter fertiger Lösung bei ≤ 6 mval, vorzugsweise bei 2-4 mval liegt.

6. Verfahren nach einem oder mehreren der Ansprüche 1 bis 5, dadurch gekennzeichnet, daß als Säure und Salz Zitronensäure, Isozitronensäure, 2-Oxoglutarsäure, Bernsteinsäure, Fumarsäure, Äpfelsäure und/oder Oxalessigsäure und deren Salze eingesetzt werden.

7. Verfahren nach einem oder mehreren der Ansprüche 1 bis 6, dadurch gekennzeichnet, daß als Säure und Salz Hydrochloride der Aminosäuren und deren Salze eingesetzt werden.

8. Lösung, insbesondere Dialyse- oder Infusionslösung, mit einer alkalisch reagierenden bicarbonathaltigen Komponente sowie einer eine Säure und/oder ein Salz umfassenden sauer reagierenden Komponente, wobei die Säure und das Salz unter Normalbedingungen als Feststoffe vorliegen, wasserlöslich und nicht hygroskopisch sind, und wobei der Gehalt an Salz und/oder Säure je Liter fertiger Lösung 6 mmol und an Bicarbonat 40 mmol nicht übersteigt.

9. Lösung nach Anspruch 8, dadurch gekennzeichnet, daß der Bicarbonatgehalt je Liter fertiger Lösung im Bereich zwischen 32 und 35 mmol liegt.

10. Lösung nach Anspruch 8 oder 9, dadurch gekennzeichnet, daß der Gehalt an Säure und/oder Salz je Liter fertiger Lösung bei ≤ 6 mval, vorzugsweise bei 2-4 mval liegt.

11. Lösung nach einem oder mehreren der Ansprüche 8 bis 10, dadurch gekennzeichnet, daß die Säure und das Salz gemäß der Ansprüche 6 und 7 ausgewählt sind.

12. Vorlagesystem zur Herstellung einer Lösung, insbesondere einer Dialyse- oder Infusionslösung, mit wenigstens zwei Vorlagebehältnissen, wobei in einem der Vorlagebehältnisse eine sauer reagierende und in dem anderen Vorlagebehältnis eine alkalisch reagierende bicarbonathaltige Komponente aufgenommen ist, wobei die sauer reagierende Komponente eine feste Säure und/oder ein festes Salz aufweist, die wasserlöslich und nicht hygroskopisch sind und die eine derartige Pufferkapazität aufweisen, daß bei einer Konzentration der Säure und/oder des Salzes von ≤ 6 mmol je Liter fertiger Lösung und einer Bicarbonat-Konzentration von bis zu 40 mmol je Liter fertiger Lösung ein physiologischer pH-Wert der fertigen Lösung einstellbar ist.

13. Vorlagesystem nach Anspruch 12, dadurch gekennzeichnet, daß die Säure und das Salz gemäß der Ansprüche 6 und 7 ausgewählt sind.

14. Vorlagesystem nach Anspruch 12 oder 13, dadurch gekennzeichnet, daß die alkalisch reagierende bicarbonathaltige Komponente in dem Vorlagebehältnis als Feststotf vorliegt.

15. Vorlagesystem nach einem oder mehreren der Ansprüche 12 bis 14, dadurch gekennzeichnet, daß drei Vorlagebehältnisse vorgesehen sind, wobei das erste Vorlagebehältnis die sauer reagierende Komponente, das zweite Vorlagebehältnis die alkalisch reagierende bicarbonathaltige Komponente und das dritte Vorlagebehältnis Zusätze enthält.

16. Vorlagesystem nach Anspruch 15, dadurch gekennzeichnet, daß alle Komponenten und Zusätze in den Vorlagebehältnissen als Feststoffe vorliegen.

17. Vorlagesystem nach Anspruch 15 oder 16, dadurch gekennzeichnet, daß die Zusätze NaCl umfassen.
